# FASCICULE DE BREVET EUROPEEN

(11) **EP 2 945 264 B1**
(45) Date de publication et mention de la délivrance du brevet: **21.09.2016**
(21) Numéro de dépôt: 14167987.8
(22) Date de dépôt: 12.05.2014
(51) Int. Cl.: H02K 7/075, A61M 39/28, H02K 26/00

(54) **Actionneur bi-position et dispositif de pince avec l'actionneur**
Stellglied mit zwei Positionen, und Zangenvorrichtung zur Verbindung mit dem Stellglied
Two-position actuator and clamping device with the actuator

(43) Date de publication de la demande: 18.11.2015
(73) Titulaire: Société Industrielle de Sonceboz S.A., 2605 Sonceboz (CH)
(72) Inventeur: Poinceau, Hubert, 2502 Bienne (CH); Martin, Thierry, 25120 Mont de Vougney (FR); Mellere, Cédric, 25190 Soulce-Cernay (FR); Wind, Hans Maarten, 2605 Sonceboz (CH); Foucaut, Antoine, 70190 Montarlot Les Rioz (FR)
(74) Mandataire: reuteler & cie SA

(56) Documents cités:
- EP-A1- 2 314 344
- WO-A1-2010/023913

## Description

La présente invention concerne un actionneur commutable entre deux positions. L'invention concerne aussi plus spécifiquement un dispositif de pince tuyau, notamment pour des appareils médicaux, intégrant un actionneur à deux positions.

Pour certaines applications, notamment des dispositifs de pince tuyau (en anglais « tube clamps ») dans le domaine médical, on utilise un actionneur commutable entre deux positions : ouverte (tuyau non-pincé) et fermée (tuyau pincé).

L'une des utilisations spécifiques de dispositifs de pince tuyau est pour des appareils médicaux transportant du sang lorsque l'appareil agit comme coeur artificiel exogène, ou pour des applications d'administration d'un liquide ou médicament. Dans ces applications, il faut pouvoir fermer le tuyau afin d'arrêter la circulation de liquide, que ce soit de manière prévisible et contrôlée, ou lorsque l'arrêt immédiat de circulation de fluide est nécessaire. Ce dernier cas peut surgir lorsqu'il y a une bulle d'air détectée dans le sang ou d'autres liquides administrés dans le corps, ces bulles d'air représentant un risque pour le patient. Le dispositif de pince tuyau doit donc pouvoir agir de manière très rapide avec une grande fiabilité. Dans ces applications, l'on doit également assurer une force d'appui de la pince qui est relativement élevée afin d'assurer que le tuyau est bien fermé, tenant compte des différents matériaux et types de tuyaux que l'on peut trouver dans ces applications. L'on doit pouvoir également facilement installer le tuyau dans le dispositif de pince tuyau, et l'enlever à la fin de l'opération. Les dispositifs de pince tuyau peuvent aussi trouver des usages dans d'autres domaines où il y a un avantage à couper l'alimentation d'un liquide dans un tuyau sans utiliser de valve ou de vanne installée dans le circuit du liquide.

Le document EP 2 314 344 A1 divulgue un dispositif pince tuyau. Les dispositifs de pince tuyau utilisés dans les dispositifs médicaux conventionnels comprennent des actionneurs linéaires avec des solénoïdes. L'un des problèmes de ces solénoïdes est qu'ils doivent être alimentés en courant dans les positions ouverte ou fermée. Ils ont donc un mauvais rendement et génèrent de la chaleur qui nécessite, dans certaines applications, de refroidir l'appareil dans lequel ils sont installés. La mise sous tension en continu peut également réduire la durée de vie et la fiabilité de ces composants. La performance de ces solénoïdes en termes de rapidité et de pression de pincement n'est également pas optimale. L'avantage des actionneurs à deux positions basés sur des solénoïdes est leur faible coût de revient. Il y aurait toutefois un avantage à remplacer ces systèmes par des dispositifs moins gourmands et plus performants mais qui sont également compacts et économes.

Un objet général de l'invention est de fournir un actionneur commutable entre deux positions qui est performant, fiable et qui consomme peu d'énergie.

Pour une application dans le domaine médical, un objet spécifique de l'invention est de fournir un actionneur pour un dispositif de pince tuyaux qui est performant, fiable et qui consomme peu d'énergie.

Il est avantageux de fournir un actionneur commutable entre deux positions qui est rapide.

Il est avantageux de fournir un actionneur économique à fabriquer et à installer.

Dans la présente invention, on décrit un actionneur comprenant un moteur électrique avec un axe de sortie rotatif et un organe de sortie commutable entre deux positions angulaires fixé à l'axe de sortie. L'actionneur comprend un mécanisme de bascule comprenant un ressort et un bras mobile non rectiligne. Le ressort est fixé d'une part au bras mobile et d'autre part à une ancre de ressort fixe sur un support fixe. Le bras mobile est couplé au moyen d'un pivot de manière pivotante au support de l'organe de sortie. La forme non rectiligne du bras mobile est configurée pour permettre au bras mobile de partiellement entourer l'axe de sortie du moteur électrique dans une desdites deux positions.

Une ligne virtuelle formée entre les centres desdits pivot du mécanisme de bascule et l'ancre de ressort fixe correspondant à une résultante de la force de traction sur le bras mobile. Dans une forme d'exécution le mécanisme de bascule est mono-stable. Dans la variante mono-stable, lors du pivotement de l'organe de sortie d'une première position à une deuxième position, la ligne virtuelle passe d'une position distant de l'axe de rotation à une position proche de l'axe de rotation, la ligne virtuelle ne traversant pas l'axe de rotation.

Dans une autre forme d'exécution, le mécanisme de bascule est bi-stable. Dans la variante bi-stable, le mécanisme de bascule est configuré pour que ladite ligne virtuelle, lors du pivotement de l'organe de sortie d'une première position à une deuxième position, fait passer la ligne virtuelle d'une position distant de l'axe de rotation à une position de l'autre côté de l'axe de rotation, la ligne virtuelle traversant l'axe de rotation.

Selon une forme d'exécution, le bras mobile a une forme essentiellement courbe et l'ancre de ressort mobile respectivement le pivot sont disposés essentiellement à des extrémités opposées du bras mobile.

Selon une forme d'exécution, le moteur électrique est un moteur couple brushless monophasé.

Selon une forme d'exécution, l'organe de sortie est un organe de pince et comprend une extension de pince s'étendant d'un support et disposé à une distance non nulle d'un axe de rotation de l'organe de sortie.

Selon une forme d'exécution, l'organe de sortie entraîne un système linéaire. L'organe de sortie peut comprendre une came engageant un organe à déplacement linéaire

Dans la présente invention, on décrit aussi un dispositif de pince comprenant l'actionneur et un dispositif support de tuyau comprenant un corps guide tuyau avec un élément d'appui, et un mécanisme de verrouillage configuré pour tenir un tuyau souple contre l'élément d'appui. Une extension de pince de l'organe de sortie de l'actionneur est configurée pour pincer le tuyau fermé contre l'élément d'appui dans la position fermée.

Le dispositif peut comprendre en outre un organe d'ouverture de pince couplé au levier pivotant et configuré pour engager l'organe de sortie lorsque le levier est tourné de sa position verrouillée à sa position ouverte afin de basculer l'organe de sortie de sa position fermée à sa position ouverte. L'organe d'ouverture de pince peut être configuré pour pouvoir se découpler de l'organe de sortie afin que ce dernier puisse bouger librement sans entrainer ledit organe d'ouverture de pince.

Selon une forme d'exécution, l'organe d'ouverture de pince comprend une ou plusieurs dents configurées pour engager une ou plusieurs dents du support de l'organe de sortie.

Selon une forme d'exécution, l'organe de sortie comprend des butées coopérant avec un arrêt sur le corps guide tuyau pour définir les positions extrêmes de fermeture et d'ouverture.

Selon une forme d'exécution, le dispositif peut comprendre deux actionneurs disposés de sorte à ce que leurs extensions de pince sont disposées de côté opposé d'un élément d'appui configuré pour l'installation de deux tuyaux souples.

Selon une forme d'exécution, l'organe d'ouverture de pince est solidaire du levier pivotant.

Selon une forme d'exécution, un capteur de position absolu est intégré dans le dispositif, le capteur de position configuré pour détecter la position de l'axe, du support ou de l'extension de pince.

Selon une forme d'exécution, le dispositif comprend une réserve locale d'énergie électrique permettant de transformer un système mécaniquement bistable en un système monostable (failsafe) à consommation de courant quasiment nulle dans les deux positions.

Dans un aspect de l'invention, l'actionneur du dispositif pince-tuyau est bistable. Cela permet de garantir la force ou le couple dans les deux positions stables, sans consommation de courant. Le passage d'une position à l'autre se faisant en alimentant l'actionneur pendant un court instant. Il y a plusieurs variantes d'actionneur bistable. Dans une variante on peut utiliser des forces magnétiques, avec un design particulier du circuit principal ou avec l'ajout d'un aimant auxiliaire. Dans une autre variante on utilise un ressort. Dans cette dernière variante, pour les mouvements rotatifs, le trajet du ressort de traction coupe virtuellement l'axe de rotation du moteur de l'actionneur. Le ressort est disposé à l'une des deux extrémités de l'axe de sortie rotatif.

Une solution proposée, applicable notamment pour les mouvements rotatifs, vise à intégrer le ressort nécessaire à la fonction bistable à un endroit quelconque du dispositif. Pour cela, un levier est solidaire en rotation de l'axe en mouvement. Sur ce levier est monté un pivot permettant la rotation d'un bras mobile de forme arrondie. Une extrémité de ce bras mobile est donc en rotation autour de ce pivot, l'autre extrémité étant fixée au ressort. La forme arrondie du bras mobile permet de passer autour de l'axe et le ressort de traction peut alors produire un couple positif ou négatif selon la position. Avec cette solution, le trajet du ressort ne coupe plus l'axe du dispositif rotatif.

Le ressort possède bien deux positions pour lesquelles sa longueur est minimale et le dispositif est stable, séparées par une zone d'élongation instable qui peut être parcourue soit grâce à l'alimentation de l'actionneur, soit par un dispositif manuel supplémentaire.

D'autres buts et aspects avantageux de l'invention apparaitront à la lecture de la description détaillée de formes d'exécution et des dessins.

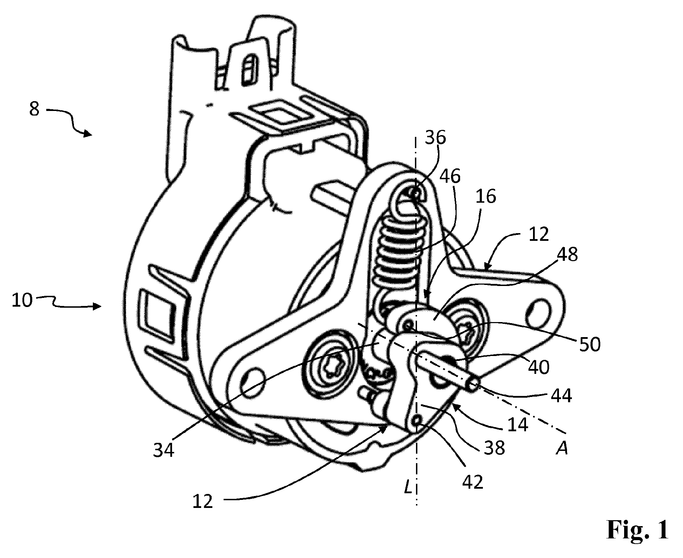

Les Fig. 1 est une vue en perspective d'un actionneur commutable entre deux positions selon une forme d'exécution de l'invention ;
La Fig. 2 est une vue en perspective d'un dispositif de pince tuyaux selon une forme d'exécution de l'invention ;
La Fig. 3a est une vue en perspective d'un dispositif de pince tuyaux selon une deuxième forme d'exécution de l'invention ;
La Fig.3b est une vue en perspective d'un dispositif de pince tuyaux selon une troisième forme d'exécution de l'invention ;

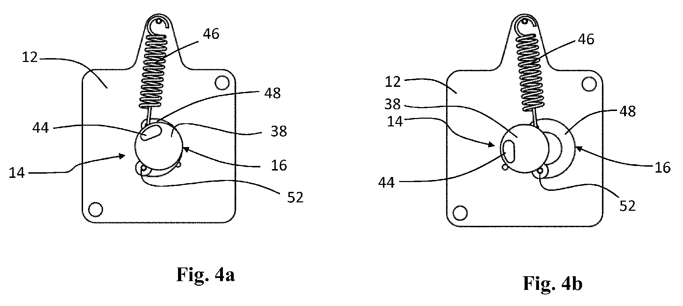

Les Fig. 4a et 4b sont des vues schématiques d'un actionneur commutable entre deux positions selon une forme d'exécution de l'invention illustrant les deux positions stables de l'actionneur ;
Les Fig. 5a à 5d sont des vues schématiques d'un actionneur commutable entre deux positions selon une forme d'exécution de l'invention illustrant le déplacement entre deux positions stables de l'actionneur ;
Les Fig. 6a à 6c sont des vues schématiques d'un dispositif de pince tuyaux selon une autre forme d'exécution de l'invention, illustrant le déplacement d'une garde tuyau de l'actionneur ;
Les Fig. 7 et 8 sont des vues schématiques d'un dispositif de pince tuyau selon des formes d'exécution de l'invention avec une sortie linéaire.

Faisant référence aux figures, un dispositif de pince 4, 4', 4" pour l'ouverture et la fermeture d'un tuyau 2 par pincement du tuyau est illustré. Le dispositif de pince comprend un dispositif support de tuyau 6 et un ou plusieurs actionneurs 8, Le dispositif support de tuyau 6 comprend un corps guide tuyau 18 et un mécanisme de verrouillage 20, 20'.

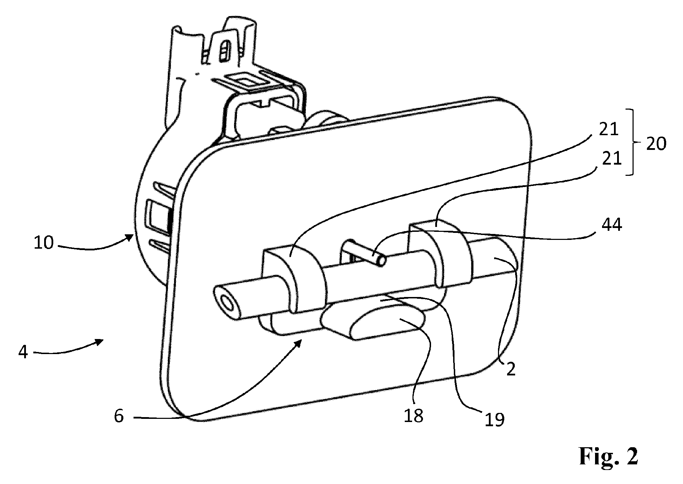

Dans la forme d'exécution illustrée à la figure 2, le corps guide tuyau 18 comprend un élément d'appui 19 sur lequel une section du tuyau 2 repose, l'élément d'appui 19 formant un appui contre une force de pincement appuyant sur cette section de tuyau 2 par un organe de sortie de l'actionneur 14. Le mécanisme de verrouillage 20 dans cette forme d'exécution comprend au moins un crochet 21, de préférence au moins deux crochets 21 disposés de part et d'autre de l'élément d'appui 19 dans la direction d'extension du tuyau souple 2. Dans cette variante, les surfaces d'appui des crochets 21 sont opposées à la surface d'appui de l'élément d'appui 19. Le tuyau 2 peut être inséré dans les crochets 21 en pliant le tuyau 2 autour de l'élément d'appui 19 et en remontant les deux portions de tuyau 2 de part et d'autre de l'élément d'appui 19 jusqu'à ce que ces portions s'insèrent dans les crochets 21. L'espacement de l'ouverture des crochets 21 peut être légèrement plus faible que le diamètre du tuyau 2 destiné à être installé, de sorte à ce que le tuyau 2 soit retenu dans le crochet 21 par un effet de léger pincement, l'élasticité nécessaire pour l'insertion du tuyau 2 dans le crochet provenant essentiellement de la souplesse du tuyau 2. Le dispositif support tuyau 6 peut être intégré dans un dispositif médical, notamment une face externe du dispositif médical accessible par un opérateur du dispositif pour l'installation du tuyau 2.

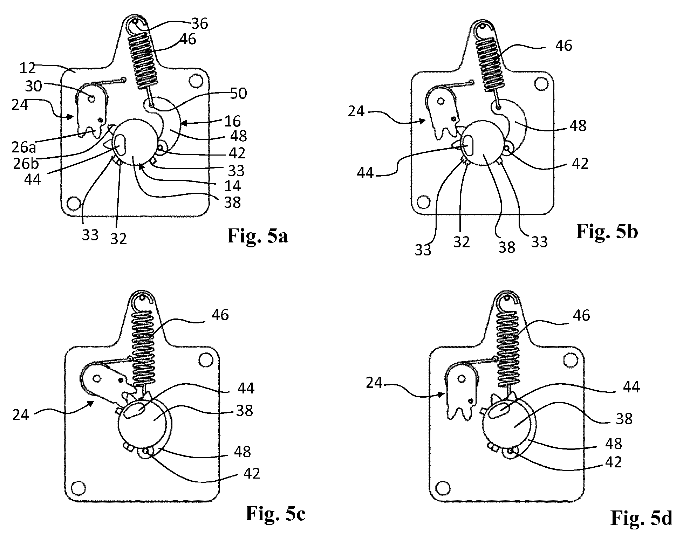

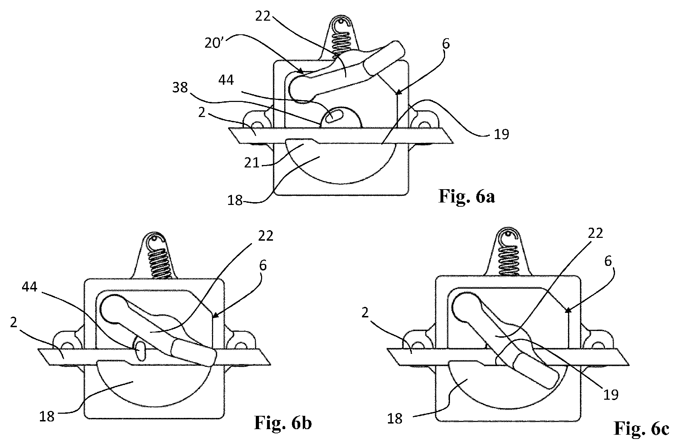

Dans une deuxième variante, illustrée dans les figures 6a à 6c, le mécanisme de verrouillage 20' du tuyau 2 comprend un levier pivotant 22 permettant le positionnement du tuyau souple 2 sur l'élément d'appui 19 lorsque le levier 22 est pivoté dans sa position ouverte tel qu'illustré dans la figure 6a, et qui peut être pivoté dans une position fermée tel qu'illustré dans les figures 6b et 6c en verrouillant le tuyau souple 2 sur l'élément d'appui 19. Dans la position fermée, le levier pivotant 22 croise l'élément d'appui 19 en bloquant un déplacement latéral du tuyau souple 2. Le levier pivotant 22 comprend également une épaule interne (non-illustré) qui vient en appui contre le tuyau souple 2 en position opposée à l'élément d'appui 19 afin de bloquer le tuyau souple 2 contre l'élément d'appui 19. Le levier pivotant 22 est couplé à un organe d'ouverture de pince 24 illustré dans les figures 5a à 5d.

L'actionneur 8 comprend un moteur électrique 10 avec un axe de sortie 34 rotatif, une bride 12, un organe de sortie 14, et un mécanisme de bascule 16. Le moteur électrique est un moteur électrique rotatif avec un stator et un rotor couplé à l'axe de sortie 34. Le moteur électrique peut être de différents types, tel qu'un moteur DC sans contacts (« brushless »), un moteur pas-à-pas, ou encore d'autres types de moteurs électriques tournants connus en soi. Dans une forme d'exécution préférée, le moteur électrique est un moteur couple brushless monophasé. Ce moteur présente l'avantage d'un couple résiduel, c'est-à-dire sans courant, faible et d'un couple proportionnel au courant d'alimentation sensiblement constant sur toute la course lorsque le circuit magnétique n'est pas saturé. La direction du couple est fonction de la polarité d'alimentation et ces actionneurs peuvent avoir une course limitée (typiquement de 75 à 80°), qui se prête bien à une application de commutation entre deux positions, par exemple pour un dispositif de pince tuyau.

Un capteur de position (non-illustré) peut être intégré dans l'actionneur, par exemple un capteur magnétique sans contact indiquant une position ou valeur absolue. La présence d'un capteur de position absolue apporte une sécurité de fonctionnement élevée. Cela permet notamment, en fixant les seuils appropriés à la dimension et à la souplesse du tuyau, de détecter les positions ouverte et fermée et, dans la position fermée, de détecter s'il y a une position fermée sans tuyau ou une position fermée avec tuyau. Dans une forme d'exécution, le capteur de position peut être utilisé pour un asservissement en boucle fermée, cela permettant d'optimiser la consommation électrique, le temps de déplacement, la force de fermeture ou le bruit du dispositif.

L'actionneur ou le dispositif de commande peuvent également comprendre un stock d'énergie locale, par exemple sous forme d'une pile ou d'un condensateur, associé à un élément de commutation permettant de transformer un actionneur à deux positions stables (bi-stable) en un actionneur bi-stable ayant une position de sécurité dite "failsafe". Cela permet de combiner l'avantage d'une faible consommation électrique du système bi-stable avec la sécurité d'un système mono-stable. En effet, dans un système bi-stable le stock d'énergie locale permet de donner une impulsion électrique uniquement pour passer d'une position à une autre lorsqu'il y a une coupure de courant pour que le système retrouve une position de sécurité correspondant à une position de repos connue et garantie en l'absence d'alimentation électrique.

La bride 12 permet de fixer l'actionneur 8 à un dispositif support de guide tuyau et/ou à un appareil médical, et optionnellement de fournir un élément de fixation telle qu'une ancre de ressort fixe 36. L'ancre de ressort fixe 36 peut toutefois aussi être disposée sur tout autre support statique par rapport au moteur 10.

L'organe de sortie 14 comprend un support 38 fixé à l'axe de sortie 34 du moteur électrique. Le support 38 peut par exemple comprendre un orifice 40 configuré pour fixer le support sur l'axe de sortie 34, par exemple en chassant le support sur l'axe. Dans le cadre de l'invention, beaucoup d'autres fixations entre l'axe de sortie et le support peuvent bien entendu être envisagées, les deux pièces pouvant être soudées ensemble, collées, ou faisant partie d'une pièce intégralement formée. L'organe de sortie 14 comprend en outre un pivot 42 pour un bras mobile 48 du mécanisme de bascule 16, par exemple sous forme d'un axe, disposé à une certaine distance de l'axe de sortie 34 et du support 38. L'axe peut être fixé au support 38, ou à un bras mobile 48 du mécanisme de bascule. L'organe de sortie 14 comprend en outre une extension de pince 44 destinée à venir en appui contre le tuyau souple et pour le pincer jusqu'à ce qu'il soit fermé, l'extension de pince 44 étant disposée à une certaine distance de l'axe de rotation A du support 38.

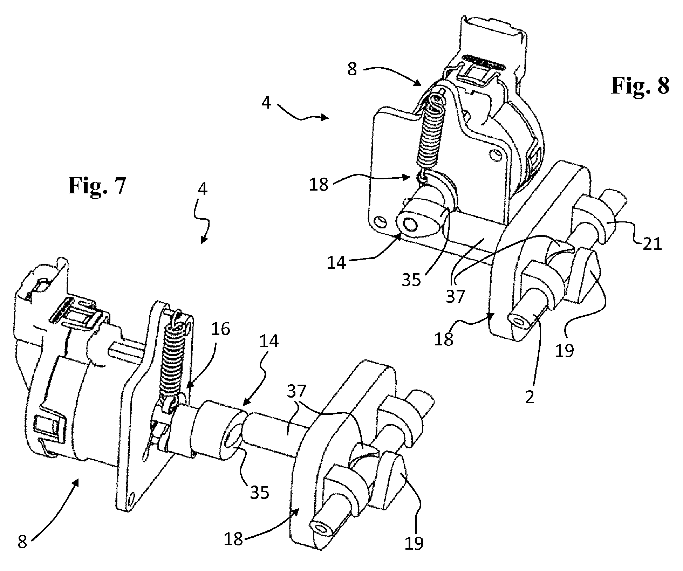

Dans une réalisation particulière, l'axe 34 ou le support 38 peuvent entraîner un dispositif linéaire ou pseudo linéaire faisant office d'extension de pince. Selon les besoins, le couplage rotatif-linéaire peut se faire avec un mécanisme bielle-manivelle, un levier, une came radiale ou axiale, ou tout autre moyen connu en soi. Figure 7 illustre un dispositif de pince selon une forme d'exécution de l'invention où l'organe de sortie 14 comprend un came qui effectue une transformation de mouvement rotatif en un mouvement linéaire sur une extension de pince 37 selon l'axe du moteur. Figure 8 illustre un dispositif de pince selon une forme d'exécution de l'invention où l'organe de sortie 14 comprend un came qui effectue une transformation de mouvement rotatif en un mouvement linéaire sur une extension de pince 37 orthogonal à l'axe de rotation du moteur.

Le mécanisme de bascule 16 comprend un bras mobile 48 non rectiligne et un ressort 46. Le bras mobile 48 est couplé de manière pivotante au pivot 42 de l'organe de sortie 14. Le bras mobile 48 comprend une ancre de ressort mobile 50 et est également couplé au ressort 46. Le ressort 46 est fixé d'une part à l'ancre de ressort mobile 50 disposée à une extrémité du bras mobile 48 et d'autre part à l'ancre de ressort fixe 36 sur la bride 12 ou à un autre support fixe par rapport au moteur électrique 10. Le ressort 46 est configuré pour appliquer une force de tension sur le bras mobile 48 au point de l'ancre de ressort mobile 50 vers l'ancre de ressort fixe 36. Par rapport à un plan virtuel orthogonal à la force de traction du ressort et traversant l'axe de rotation A, dans une forme d'exécution le pivot 42 se trouve d'un côté dudit plan virtuel orthogonal, distant de l'ancre de ressort fixe 36, et l'ancre de ressort mobile 50 disposée sur le bras mobile 48 se trouve de l'autre côté du plan virtuel orthogonal du même côté que l'ancre de ressort fixe 36.

Le bras mobile 48 non rectiligne peut avoir, dans une forme d'exécution, une forme généralement courbe permettant au bras mobile 48 de prendre une position contournant en partie l'axe de sortie 34. Cela permet à une ligne virtuelle *L* formée entre le pivot 42 et l'ancre de ressort fixe 36 de se trouver d'un côté de l'axe de rotation *A* lorsque le bras mobile se trouve dans une première position, et lorsque le bras mobile se trouve dans une deuxième position, de se trouver d'un autre côté de l'axe de rotation *A.* En d'autres termes, lorsque le bras mobile 48 se déplace d'une première position à une deuxième position, la ligne virtuelle *L* formée entre le pivot 42 et l'ancre de ressort fixe 36 coupe l'axe de rotation A. La force de tension appliquée par le ressort 46 sur le bras mobile 48 génère donc dans cette forme d'exécution deux positions stables, l'actionneur étant par conséquent bi-stable.

Le bras mobile 48 peut avoir une forme essentiellement courbe et le pivot 42 respectivement l'ancre de ressort mobile 50 peuvent se trouver à des extrémités opposées du bras mobile. Toutefois, dans le cadre de l'invention le bras mobile 48 peut avoir d'autres formes et peut supporter d'autres composants par exemple le bras mobile peut supporter un élément d'un capteur de position coopérant avec un élément de capteur de position sur la bride ou sur le moteur (par exemple l'axe de sortie ou sur le support de l'organe de sortie). Afin d'obtenir les positions bi-stables, la forme du bras mobile 48 doit être configurée de manière non rectiligne de sorte à faire passer la ligne virtuelle *L* formée entre le pivot 42 et l'ancre de ressort fixe 36 d'un côté de l'axe de la rotation A à l'autre côté à savoir que cette ligne traverse l'axe de rotation *A* lors du basculement d'une position à l'autre position.

Dans le cadre de l'invention, une variante mono-stable est également possible. Dans la variante mono-stable, l'axe de rotation *A* n'est pas traversé par la ligne virtuelle entre le pivot 42 et l'ancre de ressort fixe 36. Dans une configuration mono-stable, la ligne virtuelle L peut, dans une deuxième position, être alignée avec l'axe de rotation A ou être très proche de l'axe de rotation par rapport à une première position, de sorte à ce qu'en cas de coupure de courant cette deuxième position est instable et permet à l'actionneur 8 de retourner à la première position due à la force de traction appliquée par le ressort 46. En raison de la force de traction appliquée par le ressort 46 sur le bras mobile dans la deuxième position, correspondant à la position ouverte du dispositif de pince 24, le couple pour maintenir l'actionneur 8 dans la position ouverte est très faible. La variante mono-stable peut donc être configurée pour être très économe en énergie électrique lorsque du fluide circule dans le tuyau 2. En cas de coupure de courant, l'organe de sortie 14 retourne à la première position, correspondant dans cet exemple à une position où le tuyau 2 est fermé. Le retour de l'organe de sortie 14 peut être effectué uniquement par la force de traction du ressort 46 dans le cas d'une variante mono-stable, le cas échéant on peut effectuer une très faible impulsion, lorsque la ligne virtuelle *L* se trouve sur ou très proche de l'axe de rotation *A.*

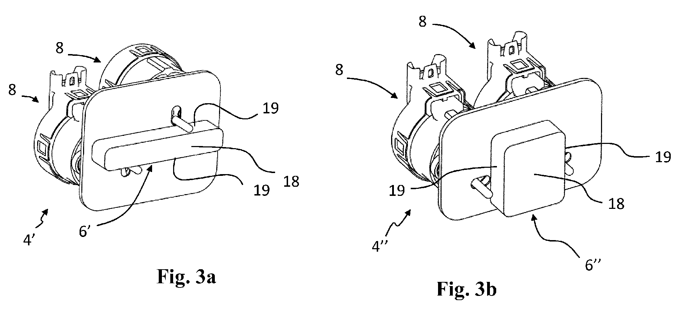

Faisant référence aux figures 3a et 3b, il est également possible selon des formes d'exécution d'avoir un dispositif de pince configuré pour pincer deux tuyaux 2, par exemple ce dispositif étant muni de deux actionneurs 8 comprenant deux moteurs électriques 10 montés à un dispositif support de tuyau 6', 6". Dans ces configurations, l'élément d'appui 19 peut être disposé entre les extensions de pince 44 de deux actionneurs, ces extensions de pince agissant donc dans des sens contraires. Dans cette configuration, il peut s'agir de deux tuyaux d'alimentation de liquide, ou d'un tuyau d'alimentation et d'un tuyau de retour d'un circuit fermé afin de pouvoir fermer simultanément le tuyau d'alimentation et le tuyau de retour.

Faisant référence aux figures 5a à 5d et 6a à 6c dans une forme d'exécution le mécanisme de verrouillage 20' comprend un levier pivotant 22 couplé à un organe d'ouverture de pince 24. L'organe d'ouverture de pince 24 comprend une ou plusieurs dents 26a configurées pour engager des dents 26b s'étendant de l'organe de sortie 14 et notamment du support 38 de l'organe de sortie 14. Le pivot 30 autour duquel l'organe d'ouverture de pince 24 pivote correspond au pivot du levier pivotant 22. L'organe d'ouverture de pince 24 peut être intégralement formé avec le levier, par exemple sous forme d'une pièce injectée en plastique, ou comprendre une pièce séparée fixée au levier 22. L'organe d'ouverture de pince 24 pivote donc avec le levier pivotant 22.

La figure 5a illustre l'organe d'ouverture de pince 24 dans une position désengagée dans laquelle les dents 26a, 26b sont désengagées. Dans cette position, le levier 22 est dans la position fermée, tel qu'illustré dans la figure 6c où le tuyau est pincé. La figure 5a correspond à une position stable, cette position correspondant aussi à une position de sécurité dans une application où l'on souhaite avoir le tuyau 2 fermé lorsqu'il y a un défaut telle qu'une coupure de courant. Lorsqu'un opérateur veut dégager le tuyau 2 du dispositif de pince, il actionne manuellement le levier pivotant 22 en le tournant de la position fermée à la position ouverte tel qu'illustré dans la figure 6a. Lors de ce basculement de la position fermée à la position ouverte, l'organe d'ouverture de pince 24 pivote tel qu'illustré dans les figures 5b et 5c et les dents 26a de l'organe d'ouverture de pince 14 engagent les dents 26b du support 38 de l'organe de sortie 14 le faisant tourner de la position fermée à la position ouverte (position fermée 5a, 5b, position ouverte 5c). Dans cette position ouverte, tel qu'illustré dans la figure 5c le tuyau peut être dégagé du dispositif de pince et un autre tuyau 2 peut être mis dans le dispositif de pince.

Lorsqu'un nouveau tuyau est placé dans le dispositif de pince, à savoir sur l'élément d'appui 19, le levier pivotant 22 peut ensuite être pivoté dans la position fermée dans laquelle l'extension de pince 44 appuie sur le tuyau et le ferme. Lors du basculement du levier pivotant 22 de la position ouverte à la position fermée, les dents 26a de l'organe d'ouverture de pince 24 engagent les dents 26b de l'organe de sortie 14 le faisant tourner vers la position fermée jusqu'à ce que les dents se désengagent tel qu'illustré dans la figure 5a. Le point de désengagement peut être configuré de sorte à ce que le bras mobile 48 se trouve toujours avec la ligne virtuelle *L* entre le pivot 42 et l'ancre de ressort fixe 36 du même côté de l'axe de rotation *A* que dans la position fermée de sorte à ce que l'organe de sortie 14 rebascule vers la position fermée tel qu'illustré dans les figures 5a et 5b. On peut donc alors fonctionner normalement via la commande électrique du moteur, la position d'ouverture de la pince illustrée à la figure 5d se faisant via la commande électrique du moteur.

Le désengagement de l'organe d'ouverture de pince permet, en fonctionnement (en dehors des phases de chargement/déchargement utilisant le levier), de passer d'une position à l'autre, grâce au moteur, sans faire bouger le levier 22. Le tuyau 2 reste donc bien verrouillé et il n'y a pas d'élément mobile important coté utilisateur. Cela réduit le bruit et les risques, tout en augmentant la durée de vie des éléments mobiles. Dans une réalisation particulière, un ressort ou un autre dispositif adéquat, permet de maintenir l'organe d'ouverture de pince désengagé.

L'organe d'ouverture de pince 24 peut aussi être couplé à un petit actionneur électrique (non-illustré) configuré pour commander la rotation de l'organe d'ouverture de pince 24 entre les positions d'ouverture et de fermeture afin d'opérer l'ouverture et/ou la fermeture du levier 22 de manière motorisée.

La variante des figures 5a à 5d peut correspondre à une variante mono-stable, mais également à une variante bi-stable.

L'organe de sortie 14, et notamment son support 38, peut être muni de butées 33 coopérant avec un arrêt 32 disposé sur le corps guide du tuyau 18, les butées et l'arrêt définissant les positions angulaires extrêmes de fermeture et d'ouverture de pince.

### Liste des références dans les figures

Tuyau souple 2
dispositif de pince 4, 4', 4"
   dispositif support de tube 6, 6', 6"
      corps guide tube 18
         élément d'appui 19
         arrêt 32
      mécanisme de verrouillage 20, 20'
         crochet 21
         levier pivotant 22
         organe d'ouverture de pince 24
            dents 26
            pivot 30
         butées 33
   actionneur 8
      moteur électrique 10
         axe de sortie 34
      bride 12
         ancre de ressort fixe 36
      organe de sortie 14
         support 38
            orifice 40
         pivot 42
         extension de pince 44
         butées 33
         came 35
         extension de pince 37
      mécanisme de bascule 16
         ressort 46
         bras mobile 48
            ancre de ressort mobile 50
         pivot 42
   *A* axe de rotation / direction axiale
   L ligne virtuel entre le pivot 42 et l'ancre de ressort fixe 36

## Revendications

1. Actionneur (8, 8') comprenant un moteur électrique (10) avec un axe de sortie (34) rotatif et un organe de sortie (14) commutable entre deux positions angulaires fixé à l'axe de sortie, l'actionneur comprenant en outre un mécanisme de bascule (16) comprenant un ressort (46) et un bras mobile (48) non rectiligne, le ressort étant fixé d'une part au bras mobile et d'autre part à une ancre de ressort fixe (36) sur un support fixe (12) relatif au moteur électrique, le bras mobile (48) étant couplé au moyen d'un pivot (42) de manière pivotante au support (38) de l'organe de sortie, une ligne virtuelle (L) formée entre les centres desdits pivot du mécanisme de bascule et l'ancre de ressort fixe correspondant à une résultante de la force de traction sur le bras mobile, la forme non rectiligne du bras mobile (48) configurée pour permettre au bras mobile de partiellement entourer l'axe de sortie (34) du moteur électrique dans une desdites deux positions.

2. Actionneur selon la revendication précédente, **caractérisé en ce que** le mécanisme de bascule est mono-stable, le mécanisme de bascule étant configuré pour que ladite ligne virtuelle (*L*), lors du pivotement de l'organe de sortie (14) d'une première position à une deuxième position, fait passer la ligne virtuelle (*L*) d'une position distant de l'axe de rotation (*A*) à une position proche de l'axe de rotation (*A*), la ligne virtuelle (*L*) ne traversant pas l'axe de rotation.

3. Actionneur selon la revendication 1, **caractérisé en ce que** le mécanisme de bascule est bi-stable, le mécanisme de bascule étant configuré pour que ladite ligne virtuelle (*L*), lors du pivotement de l'organe de sortie (14) d'une première position à une deuxième position, fait passer la ligne virtuelle (*L*) d'une position distant de l'axe de rotation (*A*) à une position de l'autre côté de l'axe de rotation, la ligne virtuelle (*L*) traversant l'axe de rotation.

4. Actionneur selon l'une des revendications précédentes, **caractérisé en ce que** le bras mobile (48) a une forme essentiellement courbe et l'ancre de ressort mobile (50) respectivement le pivot (42) se trouvent essentiellement à des extrémités opposées du bras mobile (48).

5. Actionneur selon l'une des revendications précédentes, **caractérisé en ce que** le moteur électrique est un moteur couple brushless monophasé.

6. Actionneur selon l'une des revendications précédentes, **caractérisé en ce que** l'organe de sortie est un organe de pince et comprend une extension de pince (44) s'étendant d'un support (38) et disposé à une distance non nulle d'un axe de rotation A de l'organe de sortie (14).

7. Actionneur selon l'une des revendications précédentes, **caractérisé en ce que** l'organe de sortie comprend une came (35) engageant une extension de pince (37) à déplacement linéaire.

8. Dispositif de pince (4, 4', 4") comprenant un actionneur (8, 8') selon l'une des revendications précédentes, **caractérisé en ce qu'**il comprend un dispositif support de tuyau (6, 6', 6") comprenant un corps guide tuyau (18) avec un élément d'appui (19), et un mécanisme de verrouillage (20, 20') configuré pour tenir un tuyau souple (2) contre l'élément d'appui (19), l'extension de pince (37, 44) de l'organe de sortie (14) étant configuré pour pincer le tuyau fermé contre l'élément d'appui (19) dans la position fermée.

9. Dispositif selon la revendication précédente, **caractérisé en ce qu'**il comprend en outre un organe d'ouverture de pince (24) couplé au levier pivotant (22) et configuré pour engager l'organe de sortie (14) lorsque le levier est tourné de sa position verrouillée à sa position ouverte afin de basculer l'organe de sortie (14) de sa position fermée à sa position ouverte.

10. Dispositif selon la revendication précédente, **caractérisé en ce que** l'organe d'ouverture de pince est configuré pour pouvoir se découpler de l'organe de sortie afin que ce dernier puisse bouger librement sans entrainer ledit organe d'ouverture de pince.

11. Dispositif selon la revendication précédente, **caractérisé en ce que** l'organe d'ouverture de pince (24) comprend une ou plusieurs dents (26a) configurées pour engager une ou plusieurs dents (26b) du support (38) de l'organe de sortie (14).

12. Dispositif de pince selon l'une des revendications précédentes, **caractérisé en ce que** l'organe de sortie (14) comprend des butées (33) coopérant avec un arrêt (32) sur le corps guide tuyau (18) pour définir les positions extrêmes de fermeture et d'ouverture.

13. Dispositif selon l'une des revendications précédentes, **caractérisé en ce qu'**il comprend deux actionneurs (8) disposés de sorte à ce que leurs extensions de pince (44) sont disposées de côté opposé d'un élément d'appui (19) configuré pour l'installation de deux tuyaux souples.

14. Dispositif selon l'une des revendications précédentes, **caractérisé en ce que** l'organe d'ouverture de pince (24) est solidaire du levier pivotant (22).

15. Dispositif selon l'une des revendications précédentes, **caractérisé en ce qu'**un capteur de position absolu permet de détecter la position de l'axe (34), du support (38) ou de l'extension de pince (44).

16. Dispositif selon l'une des revendications précédentes, **caractérisé en ce qu'**il comprend une réserve locale d'énergie électrique permet de transformer un dispositif mécaniquement bistable en un système monostable (failsafe) à consommation de courant quasiment nulle dans les deux positions.

## Patentansprüche

1. Stellglied (8, 8'), umfassend einen Elektromotor (10) mit einer rotierenden Ausgangswelle (34) und ein zwischen zwei Winkelpositionen umschaltbares, an der Ausgangswelle befestigtes Ausgangsorgan (14), wobei das Stellglied ferner einen Kippmechanismus (16) umfasst, der eine Feder (46) und einen ungeraden beweglichen Arm (48) umfasst, wobei die Feder zum einem am beweglichen Arm und zum anderen an einem festen Federanker (36) auf einem in Bezug zum Elektromotor festen Halter (12) befestigt ist, wobei der bewegliche Arm (48) mittels eines Zapfens (42) schwenkend am Halter (38) des Ausgangsorgans gekoppelt ist, wobei eine virtuelle Linie (*L*), die zwischen dem Zentrum des Zapfens des Kippmechanismus und dem Zentrum des festen Federankers gebildet ist, einer Resultierenden der Zugkraft auf den beweglichen Arm entspricht, wobei die ungerade Form des beweglichen Arms (48) konfiguriert ist, um dem beweglichen Arm zu ermöglichen, die Ausgangswelle (34) des Elektromotors in einer der zwei Positionen teilweise zu umgeben.

2. Stellglied nach vorangehendem Anspruch, **dadurch gekennzeichnet, dass** der Kippmechanismus monostabil ist, wobei der Kippmechanismus konfiguriert ist, damit die virtuelle Linie (L) beim Schwenken des Ausgangsorgans (14) von einer ersten Position in eine zweite Position die virtuelle Linie (L) von einer von der Rotationsachse (A) entfernten Position in eine zur Rotationsachse (A) nahen Position verlagert, wobei die virtuelle Linie (L) die Rotationsachse nicht durchquert.

3. Stellglied nach Anspruch 1, **dadurch gekennzeichnet, dass** der Kippmechanismus bistabil ist, wobei der Kippmechanismus konfiguriert ist, damit die virtuelle Linie (*L*) beim Schwenken des Ausgangsorgans (14) von einer ersten Position in eine zweite Position die virtuelle Linie (*L*) von einer von der Rotationsachse (A) entfernten Position in eine Position auf der anderen Seite der Rotationsachse (*A*) verlagert, wobei die virtuelle Linie (L) die Rotationsachse durchquert.

4. Stellglied nach einem der vorangehenden Ansprüche, **dadurch gekennzeichnet, dass** der bewegliche Arm (48) eine im Wesentlichen gekrümmte Form hat und sich der bewegliche Federanker (50) beziehungsweise der Zapfen (42) im Wesentlichen an entgegengesetzten Enden des beweglichen Arms (48) befinden.

5. Stellglied nach einem der vorangehenden Ansprüche, **dadurch gekennzeichnet, dass** der Elektromotor ein Einphasen-Brushless-Drehmomentmotor ist.

6. Stellglied nach einem der vorangehenden Ansprüche, **dadurch gekennzeichnet, dass** das Ausgangsorgan ein Klemmenorgan ist und eine Klemmenerweiterung (44) umfasst, die sich von einem Halter (38) erstreckt und in einem Abstand ungleich null von einer Rotationsachse A des Ausgangsorgans (14) angeordnet ist.

7. Stellglied nach einem der vorangehenden Ansprüche, **dadurch gekennzeichnet, dass** das Ausgangsorgan eine Kurvenscheibe (35) umfasst, die eine lineare Verlagerung einer Klemmenerweiterung (37) bewirkt.

8. Klemmenvorrichtung (4, 4', 4"), umfassend ein Stellglied (8, 8') nach einem der vorangehenden Ansprüche, **dadurch gekennzeichnet, dass** sie eine Schlauchhaltevorrichtung (6, 6', 6") umfasst, die einen Schlauchführungskörper (18) mit einem Stützelement (19) umfasst, und einen Verriegelungsmechanismus (20, 20'), der konfiguriert ist, um einen Schlauch (2) gegen das Stützelement (19) zu halten, wobei die Klemmenerweiterung (37, 44) des Ausgangsorgans (14) konfiguriert ist, um den geschlossenen Schlauch gegen das Stützelement (19) in der geschlossenen Position zu klemmen.

9. Vorrichtung nach vorangehendem Anspruch, **dadurch gekennzeichnet, dass** sie ferner ein Klemmenöffnungsorgan (24) umfasst, das an den schwenkenden Hebel (22) gekoppelt ist und konfiguriert, um das auf das Ausgangsorgan (14) einzuwirken, wenn der Hebel aus seiner verriegelten Position in seine geöffnete Position gedreht wird, um das Ausgangsorgan (14) aus seiner geschlossenen Position in seine geöffnete Position zu kippen.

10. Vorrichtung nach vorangehendem Anspruch, **dadurch gekennzeichnet, dass** das Klemmenöffnungsorgan konfiguriert ist, um sich vom Ausgangsorgan zu lösen, damit sich dieses frei bewegen kann, ohne auf das Klemmenöffnungsorgan einzuwirken.

11. Vorrichtung nach vorangehendem Anspruch, **dadurch gekennzeichnet, dass** das Klemmenöffnungsorgan (24) einen oder mehrere Zähne (26a) umfasst, die konfiguriert sind, um auf einen oder mehrere Zähne (26b) des Halters (38) des Ausgangsorgans (14) einzuwirken.

12. Klemmvorrichtung nach einem der vorangehenden Ansprüche, **dadurch gekennzeichnet, dass** das Ausgangsorgan (14) Anschläge (33) umfasst, die mit einem Stopp (32) auf dem Schlauchführungskörper (18) zusammenarbeiten, um die extremen Verschluss- und Öffnungspositionen zu bestimmen.

13. Vorrichtung nach einem der vorangehenden Ansprüche, **dadurch gekennzeichnet, dass** sie zwei Stellglieder (8) umfasst, die derart angeordnet sind, dass ihre Klemmenerweiterungen (44) auf der gegenüberliegenden Seite eines Stützelements (19) angeordnet sind, das für die Installation von zwei Schläuchen konfiguriert ist.

14. Vorrichtung nach einem der vorangehenden Ansprüche, **dadurch gekennzeichnet, dass** das Klemmenöffnungsorgan (24) mit dem schwenkenden Hebel (22) fest verbunden ist.

15. Vorrichtung nach einem der vorangehenden Ansprüche, **dadurch gekennzeichnet, dass** ein Absolutpositionssensor erlaubt, die Position der Welle (34), des Halters (38) oder der Klemmenerweiterung (44) zu ermitteln.

16. Vorrichtung nach einem der vorangehenden Ansprüche, **dadurch gekennzeichnet, dass** sie eine lokale Elektroenergiereserve umfasst, die erlaubt, eine mechanisch bistabile Vorrichtung in ein monostabiles System (failsafe) mit Stromverbrauch von quasi null in den zwei Positionen umzuwandeln.

## Claims

1. An actuator (8, 8') comprising an electric motor (10) with a rotary output shaft (34) and an output member (14) that is commutable between two angular positions fixed to the output shaft, the actuator further comprising a tilt mechanism (16) comprising a spring (46), a non-rectilinear movable arm (48), the spring being attached on the one hand to the movable arm and on the other hand to a fixed spring anchor (36) on a fixed support (12) relative to the electric motor, the movable arm (48) being coupled pivotally by means of a pivot (42) to the support (38) of the output member, a virtual line (L) formed between the centres of said pivot of the tilt mechanism and the fixed spring anchor corresponding to a resultant of the tension force on the movable arm, the non-rectilinear shape of the movable arm (48) configured to allow the movable arm to partially surround the output shaft (34) of the electric motor in one of said two positions.

2. The actuator according to the preceding claim, **characterized in that** the tilt mechanism is monostable, the tilt mechanism being configured so that said virtual line (L), during pivoting of the output member (14) from a first position to a second position, causes the virtual line (*L*) to move from a position distant from the axis of rotation (*A*) to a position on the other side of the axis of rotation (*A*), the virtual line (*L*) passing through the axis of rotation.

3. The actuator according to claim 1, **characterized in that** the tilt mechanism is bistable, the tilt mechanism being configured so that said virtual line (*L*), during pivoting of the output member (14) from a first position to a second position, causes the virtual line (*L*) to move from a position distant from the axis of rotation (*A*) to a position on the other side of the axis of rotation, the virtual line (*L*) passing through the axis of rotation.

4. The actuator according to one of the preceding claims, **characterized in that** the movable arm (48) has a shape that is essentially curved and the movable spring anchor (50), respectively the pivot (42), are located essentially at opposite ends of the movable arm (48).

5. The actuator according to one of the preceding claims, **characterized in that** the electric motor is a single-phase brushless torque motor.

6. The actuator according to one of the preceding claims, **characterized in that** the output member is a clamping member and comprises an clamp extension (44) extending from a support (38) and positioned at a non-zero distance from an axis of rotation A of the output member (14).

7. The actuator according to one of the preceding claims, **characterized in that** the output member comprises a cam (35) engaging a clamp extension (37) with linear displacement.

8. A clamping device (4, 4', 4") comprising an actuator (8, 8') according to one of the preceding claims, **characterized in that** it comprises a pipe support device (6, 6', 6") comprising a pipe guide body (18) with a support element (19) and a locking mechanism (20, 20') configured to hold a flexible pipe (2) against the support element (19), the clamp extension (37, 44) of the output member (14) being configured to clamp the closed pipe against the support element (19) in the closed position.

9. The device according to the preceding claim, **characterized in that** it further comprises a clamp opening member (24) coupled to the pivoting lever (22) and configured to engage the output member (14) when the lever is turned from its locked position into its open position so as to tilt the output member (14) from its closed position into its open position.

10. The device according to the preceding claim, **characterized in that** the clamp opening member is configured to be able to decouple from the output member so that the latter can move freely without driving said clamp opening member.

11. The device according to the preceding claim, **characterized in that** the clamp opening member (24) comprises one or more teeth (26a) configured to engage one or more teeth (26b) of the support (38) of the output member (14).

12. The clamping device according to one of the preceding claims, **characterized in that** the output member (14) comprises abutments (33) cooperating with a stop (32) on the pipe guide body (18) to define the extreme positions of opening and closure.

13. The device according to one of the preceding claims, **characterized in that** it comprises two actuators (8) positioned so that their clamp extensions (44) are positioned on opposite side of a support element (19) configured for the installation of two flexible pipes.

14. The device according to one of the preceding claims, **characterized in that** the clamp opening member (24) is integral with the pivoting lever (22).

15. The device according to one of the preceding claims, **characterized in that** an absolute position sensor makes it possible to detect the position of the shaft (34) of the support (38) or of the clamp extension (44).

16. The device according to one of the preceding claims, **characterized in that** it comprises a local reserve of electrical energy allowing a mechanically bistable device to be transformed into a monostable (failsafe) device with virtually no electrical power consumption in both positions.
